(19)

Europäisches
Patentamt
European
Patent Office
Office européen
des brevets

(11) **EP 4 586 262 A1**

(12) **EUROPEAN PATENT APPLICATION**
published in accordance with Art. 153(4) EPC

(43) Date of publication:
**16.07.2025 Bulletin 2025/29**

(21) Application number: **23880015.5**

(22) Date of filing: **07.09.2023**

(51) International Patent Classification (IPC):
*G16H 20/00* (2018.01)     *G16H 50/20* (2018.01)
*G16H 20/60* (2018.01)     *G16H 20/30* (2018.01)
*A63B 24/00* (2006.01)     *A61B 5/00* (2006.01)
*A61B 5/083* (2006.01)     *A61B 5/145* (2006.01)
*A61B 5/021* (2006.01)

(52) Cooperative Patent Classification (CPC):
**G16H 50/20; A61B 5/00; A61B 5/021; A61B 5/024;
A61B 5/083; A61B 5/145; A63B 24/00;
G16H 10/00; G16H 20/00; G16H 20/30;
G16H 20/60**

(86) International application number:
**PCT/KR2023/013452**

(87) International publication number:
**WO 2024/085435 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.10.2022 KR 20220135233**

(71) Applicant: **Samsung Electronics Co., Ltd.
Suwon-si, Gyeonggi-do 16677 (KR)**

(72) Inventors:
• **BRISTY, Ummey Habiba
Suwon-si, Gyeonggi-do 16677 (KR)**
• **FOYSAL, A S M
Suwon-si, Gyeonggi-do 16677 (KR)**
• **SULTANA, Jakia
Dhaka, 1205 (BD)**

(74) Representative: **Arnold & Siedsma
Bezuidenhoutseweg 57
2594 AC The Hague (NL)**

(54) **ELECTRONIC DEVICE AND OPERATION METHOD THEREOF**

(57)     An electronic device according to an embodiment includes at least one sensor, a memory storing one or more instructions, and one or more processors, and the one or more processors may be configured to execute the one or more instructions to measure, by using the at least one sensor, an amount of carbon dioxide generated by a user and a variation in body fat of the user, calculate a calorie burn amount of the user, based on the amount of carbon dioxide generated by the user, calculate a calorie intake amount of the user, based on the variation in the body fat of the user and the calorie burn amount of the user, and output user-customized healthcare information, based on the variation in the body fat of the user, the calorie burn amount of the user, and the calorie intake amount of the user.

FIG. 5

```
              START
                │
┌──────────────────────────────────────┐
│ MEASURE AMOUNT OF CARBON DIOXIDE PRODUCED │── 510
│     BY USER AND VARIATION IN BODY FAT     │
└──────────────────────────────────────┘
                │
┌──────────────────────────────────────┐
│   CALCULATE CALORIE BURN AMOUNT OF USER   │── 520
└──────────────────────────────────────┘
                │
┌──────────────────────────────────────┐
│  CALCULATE CALORIE INTAKE AMOUNT OF USER, │── 530
│ BASED ON CALORIE BURN AMOUNT OF USER AND  │
│        VARIATION IN BODY FAT              │
└──────────────────────────────────────┘
                │
┌──────────────────────────────────────┐
│   OUTPUT USER-CUSTOMIZED HEALTHCARE       │── 540
│            INFORMATION                    │
└──────────────────────────────────────┘
                │
               END
```

EP 4 586 262 A1

## Description

### Technical Field

The technical idea of the present disclosure relates to an electronic device, and more particularly, to an electronic device and operation method thereof for recommending user-customized healthcare information.

### Background Art

Recently, as research on wearable electronic devices among electronic devices has been actively conducted, various wearable electronic devices have been released or are expected to be released. Wearable electronic devices that are being currently released or are scheduled to be released include smartwatches, smart glasses, smart bands, and the like.

Wearable electronic devices are highly accessible because they are attached to a user's body, and are capable of providing users with various healthcare services in conjunction with or independently of mobile devices such as smartphones and tablets.

However, conventional wearable electronic devices have problems in providing user-customized healthcare services due to the inconvenience of having to receive user data required for healthcare services from the user and measurement errors in user data due to various causes. Therefore, there is a need for an electronic device capable of providing effective user-customized healthcare services by using accurately measured user data.

### Disclosure of Invention

### Solution to Problem

An electronic device according to an embodiment may include at least one sensor, a memory storing one or more instructions, and one or more processors.

According to an embodiment, the one or more processors may be configured to execute the one or more instructions to measure, by using the at least one sensor, an amount of carbon dioxide generated by a user and a variation in body fat of the user.

According to an embodiment, the one or more processors may be configured to calculate a calorie burn amount of the user, based on the amount of carbon dioxide.

According to an embodiment, the one or more processors may be configured to calculate a calorie intake amount of the user based on the variation in the body fat of the user and the calorie burn amount of the user.

According to an embodiment, the one or more processors may be configured to output user-customized healthcare information, based on the variation in the body fat of the user, the calorie burn amount of the user, and the calorie intake amount of the user.

An operation method of an electronic device, according to an embodiment, may include
measuring an amount of carbon dioxide generated by a user, a variation in body fat of the user, and a variation in body fat of the user.

According to an embodiment, the operation method of the electronic device may include calculating a calorie burn amount of the user, based on the amount of carbon dioxide.

According to an embodiment, the operation method of the electronic device may include calculating a calorie intake amount of the user based on the variation in the body fat of the user and the calorie burn amount of the user.

According to an embodiment, the operation method of the electronic device may include outputting user-customized healthcare information, based on the calorie burn amount of the user, the variation in the body fat of the user, and the calorie intake amount of the user.

According to an embodiment, there may be provided a computer-readable recording medium having recorded thereon a program for executing any one of the methods, performed by an electronic device, of outputting (or recommending) user-customized healthcare information.

### Brief Description of Drawings

FIG. 1 illustrates an example in which an electronic device is used, according to an embodiment.
FIG. 2 is a block diagram of an electronic device according to an embodiment.
FIGS. 3A and 3B illustrate examples of an electronic device according to an embodiment.

FIG. 4 is a block diagram of a sensing module included in an electronic device, according to an embodiment.

FIG. 5 illustrates an operation method of an electronic device, according to an embodiment.

FIG. 6 illustrates an operation in which an electronic device outputs user-customized diet management information, according to an embodiment.

FIG. 7 illustrates an operation in which an electronic device calculates a user's calorie intake amount, according to an embodiment.

FIG. 8 illustrates an operation method of an electronic device, according to an embodiment.

FIG. 9 illustrates an operation method of an electronic device, according to an embodiment.

FIG. 10 illustrates an operation method of an electronic device, according to an embodiment.

FIG. 11 illustrates an operation method of an electronic device, according to an embodiment.

FIG. 12 is a block diagram of a system according to an embodiment.

FIG. 13 illustrates a detailed configuration of an electronic device according to an embodiment.

**Mode for the Invention**

Terms used in the present specification will now be briefly described, and then the present disclosure will be described in detail.

As terms used in the present disclosure, general terms that are currently widely used are selected by taking into account functions in the present disclosure, but these terms may vary according to the intention of one of ordinary skill in the art, precedent cases, advent of new technologies, etc. Furthermore, specific terms may be arbitrarily selected by the applicant, and in this case, the meaning of the selected terms will be described in detail in the detailed description of a corresponding part of the disclosure. Thus, the terms used herein should be defined not by simple appellations thereof but based on the meaning of the terms together with the overall description of the present disclosure.

Throughout the specification, when a part "includes" or "comprises" an element, unless there is a particular description contrary thereto, it is understood that the part may further include other elements, not excluding the other elements. As used herein, the term "unit" or "module" indicates a unit for processing at least one function or operation and may be implemented using hardware or software or a combination of hardware and software.

Embodiments will be described more fully hereinafter with reference to the accompanying drawings so that they may be easily implemented by one of ordinary skill in the art. However, the present disclosure may be implemented in various different forms and is not limited to the embodiments described herein. In addition, parts not related to descriptions of the present disclosure are omitted to clearly explain the present disclosure in the drawings, and like reference numerals denote like elements throughout.

In an embodiments of the present specification, the term "user" refers to a person who controls a system, a function or an operation, and may include a developer, an administrator, or an installation technician.

In an embodiment of this specification, the term "electronic device" refers to an electronic device (or wearable electronic device) that may be worn on a user's body.

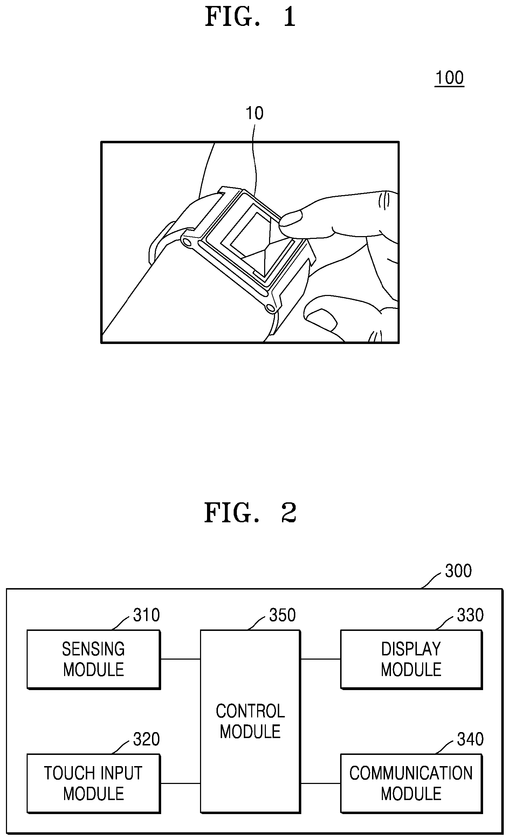

FIG. 1 illustrates an example 100 in which an electronic device is used, according to an embodiment.

Referring to FIG. 1, an electronic device 10 may be worn on a user's body to measure biometric information of the user and recommend healthcare information based on the measured biometric information.

In an embodiment, as illustrated in FIG. 1, the electronic device 10 has the shape of a watch and may be temporarily fixed to the user's wrist by a watch band or the like.

In an embodiment, the electronic device 10 may be attached to a part of the user's body to sense the biometric information of the user (e.g., the amount of carbon dioxide produced by the user, a user's blood pH, etc.) by using at least one sensor included in the electronic device 10.

According to an embodiment of the present disclosure, the electronic device may determine a user's physical condition by using the measured biometric information of the user, thereby enabling an accurate determination of the user's physical condition and providing the user with user-customized healthcare information that may effectively improve the user's health accordingly.

FIG. 2 is a block diagram of an electronic device according to an embodiment.

Components of an electronic device 300 according to an embodiment of the present disclosure are described in detail with reference to FIG. 2. The electronic device 10 of FIG. 1 may correspond to the electronic device 300 of FIG. 2.

First, according to an embodiment of the present disclosure, the electronic device 300 is a device that may be worn on a user's body and may be an electronic device that measures or provides biometric information of the user.

As illustrated in FIG. 2, according to an embodiment of the present disclosure, the electronic device 300 may include a sensing module 310, a touch input module 320, a display module 330, a communication module 340, and a control module 350.

The sensing module 310 may include one or more sensors and may detect various pieces of information for determining a user's state, a state of the electronic device 300, and a state of the surrounding environment. For example, the sensing module 310 may sense information about the user's state. The information about the user's state may include at least one of information related to the user's movement, information related to the user's location, and information related to the user's biosignal.

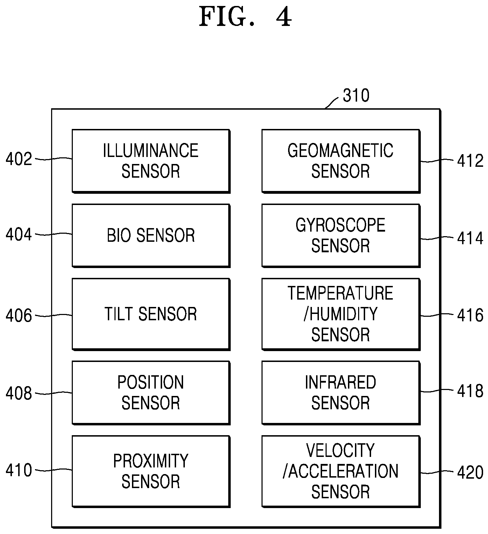

For example, the sensing module 310 may measure the amount of carbon dioxide produced by the user, a variation in the user's body fat, an electrocardiogram of the user, etc. Various sensors that may be included in the sensing module 310 are described in more detail below with reference to FIG. 4.

The touch input module 320 may receive a touch input from the user.

The touch input module 320 may detect an input from the user touching the touch input module 320 by using a pointing object. In this case, the pointing object refers to a tool for touching a touch panel via a real-touch or proximity touch. Examples of the pointing object include a stylus pen, a finger, etc.

According to an embodiment, the touch input module 320 provided in the electronic device 300 may be configured to detect not only a user's real-touch but also proximity touch on the touch input module 320.

According to an embodiment, the touch input module 320 may receive a touch input by a pointing object. The touch input may refer to a motion in which the user touches the touch input module 320 by using the pointing object and then does not move for a predetermined period of time. For example, when a touch to the touch input module 320 is continuously detected for several tens to several hundreds of milliseconds (ms), the electronic device 300 may determine that a touch input has been received.

The control module 350 may control all operations of the electronic device 300.

According to an embodiment, when a touch input is received from the touch input module 320, the control module 350 may control the sensing module 310 to measure biometric information of the user.

According to an embodiment, the control module 350 may determine the user's state based on the biometric information of the user, and control the display module 330 to display user-customized healthcare information or sleep management information based on a result of the determination.

The display module 330 may display and output information processed in the electronic device 300. The display module 320 may display at least one piece of healthcare information or sleep management information about the user transmitted from the control module 350. Furthermore, the display module 320 may further display a user interface (UI) for receiving a user input for controlling the electronic device 300 and a UI for setting parameters related to an operation of receiving a touch input and an operation of transmitting a message to a receiving device.

The display module 330 may be configured as a touch screen by forming a layer structure with the touch input module 320. In this case, the touch screen may be used as an input device as well as an output device by performing functions of both the touch input module 320 and the display module 330. The display module 330 may include at least one of a liquid crystal display (LCD), a thin-film transistor LCD (TFT-LCD), an organic light-emitting diode (OLED) display, a flexible display, a three-dimensional (3D) display, and an electrophoretic display. In addition, the display module 330 may be configured as a translucent optical waveguide (e.g., a prism).

The communication module 340 may communicate with an external electronic device or server. The communication module 340 may receive, from the external electronic device, a control signal for controlling the electronic device 300, a signal indicating information about a state of the external electronic device, etc. In addition, the communication module 340 may transmit, to the external electronic device, a control signal for controlling the electronic device 300, a signal indicating information about the state of the electronic device 300, etc.

For example, the communication module 340 may include at least one of a Bluetooth Low Energy (BLE) module, a Bluetooth module, a near field communication (NFC) module, a radio frequency (RF) module, and a mobile communication module, or a combination thereof.

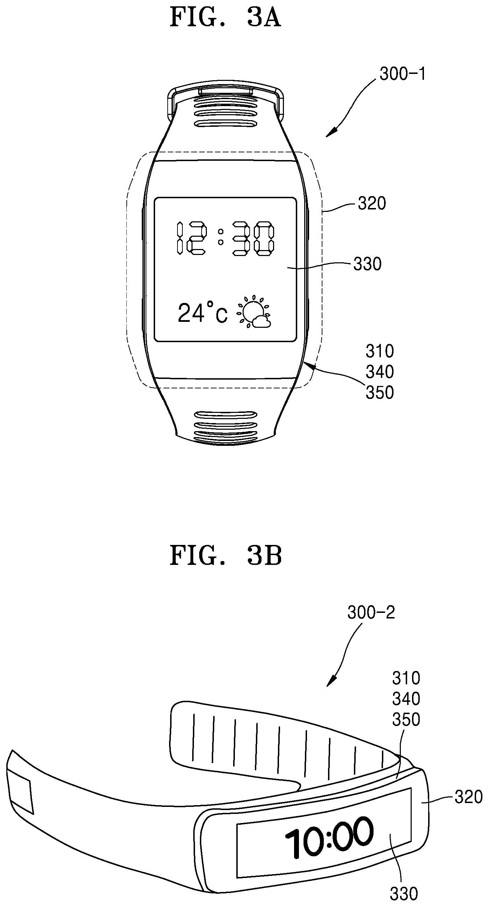

FIGS. 3A and 3B illustrate examples of an electronic device according to an embodiment.

According to an embodiment of the present disclosure, as illustrated in FIG. 3A, an electronic device 300-1 may be implemented in the form of a smartwatch that is fixed to a user's wrist. The electronic device 300 of FIG. 2 may correspond to the electronic device 300-1 of FIG. 3A or an electronic device 300-2 of FIG. 3B. In addition, the sensing module 310, the touch input module 320, the display module 330, the communication module 340, and the control module 350 of FIG. 2 may respectively correspond to a sensing module 310, a touch input module 320, a display module 330, a communication module 340, and a control module 350 of FIGS. 3A and 3B.

The electronic device 300-1 in the form of the smartwatch illustrated in FIG. 3A may include the touch input module 320, the display module 330, and the control module 350. However, all of the components shown in FIG. 3A are not essential components of the electronic device 300-1. The electronic device 300-1 may be implemented by more components than those shown in FIG. 3A, or it may be implemented by fewer components than those shown in FIG. 3A.

Some of the components included in the electronic device 300-1 may be built into the electronic device 300-1, and other components thereof may be mounted on outside of the electronic device 300-1. For example, the control module 350 may be built into the electronic device 300-1.

In addition to the control module 350, the electronic device 300-1 may further include therein the sensing module 310 that detects a state of the user wearing the electronic device 300-1, a state of the electronic device 300-1, or an external state. In addition, the electronic device 300-1 may further include therein the communication module 340 for communicating with another electronic device. The touch input module 320 and the display module 330 may be mounted on the outside of the electronic device 300-1. The components built into the electronic device 300-1 and the components mounted on the outside of the electronic device 300-1 are not limited to those described above.

A frame that maintains the shape of the electronic device 300-1 may be composed of a material such as plastic and/or metal, and include wiring that connects the components included in the electronic device 300-1 to each other.

The touch input module 320 may include a touch pad operable by a user's finger. In FIG. 3A, the touch input module 320 is illustrated as being located on a watch face included in the electronic device 300-1, but the touch input module 320 may also be located at another position on the electronic device 300-1. The electronic device 300-1 may receive various user inputs via the touch input module 320.

The display module 330 may be located on the watch face of the electronic device 300-1 as illustrated in FIG. 3A. The display module 330 may be configured as a touch screen by forming a layer structure with a touch pad that receives a touch input. In this case, the display module 330 may perform the function of the touch input module 320 together.

Referring to FIG. 3B, according to an embodiment of the present disclosure, the electronic device 300 may be configured in the form of a smart band that is fixed to the user's wrist, forearm, ankle, etc.

The electronic device 300-2 in the form of the smart band illustrated in FIG. 3B may include the touch input module 320, the display module 330, and the control module 350. However, all of the components shown in FIG. 3B are not essential components of the electronic device 300-2. The electronic device 300-2 may be implemented by more components than those shown in FIG. 3B, or it may be implemented by fewer components than those shown in FIG. 3B.

Some of the components included in the electronic device 300-2 may be built into the electronic device 300-2, and other components thereof may be mounted on outside of the electronic device 300-2. For example, the control module 350 may be built into the electronic device 300-2.

In addition to the control module 350, the electronic device 300-2 may further include therein the sensing module 310 that detects a state of the user wearing the electronic device 300-2, a state of the electronic device 300-2, or an external state. In addition, the electronic device 300-2 may further include therein the communication module 340 for communicating with another electronic device. The touch input module 320 and the display module 330 may be mounted on the outside of the electronic device 300-2. The components built into the electronic device 300-2 and the components mounted on the outside of the electronic device 300-1 are not limited to those described above.

A frame that maintains the shape of the electronic device 300-2 may be composed of a material such as plastic and/or metal, and may include an elastic band so that it may be fixed to the user's body regardless of the user's body size. In addition, the frame that maintains the shape of the electronic device 300-2 may include wiring that connects the components included in the electronic device 300-2 to each other.

The touch input module 320 may include a touch pad operable by a user's finger. In FIG. 3B, the touch input module 320 is illustrated as being located on a side on which the display module 330 is located, but the touch input module 320 may also be located at another position on the electronic device 300-2. The electronic device 300-2 may receive various user inputs via the touch input module 320.

The display module 330 may be configured as a touch screen by forming a layer structure with a touch pad that receives a touch input. In this case, the display module 330 may perform the function of the touch input module 320 together.

As illustrated in FIG. 3A and FIG. 3B, an electronic device according to an embodiment of the present disclosure may be implemented in various forms.

FIG. 4 is a block diagram of a sensing module included in an electronic device, according to an embodiment.

In detail, FIG. 4 is a block diagram of the sensing module 310 included in the electronic device 300 of FIG. 2.

Referring to FIG. 4, according to an embodiment of the present disclosure, the sensing module 310 may include at least one of an illuminance sensor 402, a biosensor 404, a tilt sensor 406, a position sensor 408, a proximity sensor 410, a geomagnetic sensor 412, a gyroscope sensor 414, a temperature/humidity sensor 416, an infrared (IR) sensor 418, and a velocity/acceleration sensor 420, or a combination thereof. In addition to the types of sensors illustrated in FIG. 4, various sensors may be included in the sensing module 310.

The sensing module 310 may include various combinations of sensors depending on an implemented configuration of the electronic device 300. For example, the electronic device 300 may sense information related to a user's movement (e.g., information about the user's activity status) as information about the user's state by including at least one or a combination of the tilt sensor 406, the position sensor 408, the geomagnetic sensor 412, the gyroscope sensor 414, and the velocity/acceleration sensor 420.

The biosensor 404 may sense information related to the user's body composition and biosignals. The biosensor 404 may include, for example, at least one of a heart rate sensor, a blood glucose sensor, a blood pressure sensor, a sweat sensor, a body temperature sensor, and an iris sensor.

For example, the biosensor 404 may measure the calorie burn amount of the user.

For example, the biosensor 404 may measure a body fat percentage or body fat amount of the user.

For example, the biosensor 404 may measure a metabolic rate of the user.

For example, the biosensor 404 may measure the user's sleep cycle or sleep period.

For example, the biosensor 404 may measure a maximum oxygen partial pressure ($VO_2MAX$) within the user's body.

For example, the biosensor 404 may measure a partial pressure of carbon dioxide in the user's body.

For example, the biosensor 404 may measure a pH level in the user's blood by using the amount of carbon dioxide produced by the user.

The position sensor 408 may include a global positioning system (GPS) module, a Wi-Fi Protected Setup (WPS) module, a BLE module, etc. For example, the position sensor 408 may include a GPS module and sense a position of the electronic device 300 by using a GPS. Alternatively, the position sensor 408 may include a WPS module and sense the position of the electronic device 300 by using a Wi-Fi map.

For example, the position sensor 408 may sense contextual information of the user based on the user's location.

The temperature/humidity sensor 416 may sense at least one of a temperature and a humidity of the surrounding environment of the electronic device 300.

For example, the velocity/acceleration sensor 420 may obtain information related to a movement of the user wearing the electronic device 300 by sensing at least one of a velocity and an acceleration of the electronic device 300.

The IR sensor 418 includes a nondispersive IR (NDIR) sensor and may perform real-time transcutaneous measurement of the amount of carbon dioxide produced by the user. For example, the NDIR sensor may measure the amount of carbon dioxide produced by the user by using the characteristic of carbon dioxide absorbing only specific light in the infrared spectrum (e.g., carbon dioxide absorption spectrum characteristics).

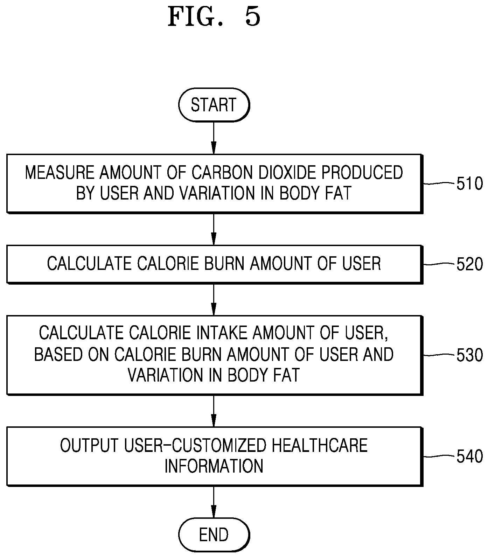

FIG. 5 illustrates an operation method of an electronic device, according to an embodiment.

In detail, FIG. 5 illustrates an operation method, performed by a processor of the electronic device 300 of FIG. 2, of outputting user-customized healthcare information by measuring the amount of carbon dioxide produced by a user.

Referring to FIG. 5, operations for outputting the user-customized healthcare information may include operations 510, 520, 530, and 540.

In operation 510, the processor may measure the amount of carbon dioxide produced by the user and a variation in the user's body fat.

According to an embodiment, the processor may transcutaneously measure the amount of carbon dioxide produced by the user by using at least one sensor (e.g., an NDIR sensor).

In an embodiment, the processor may measure the amount of carbon dioxide produced by the user based on Equation 1 below.

[Equation 1]

$$\text{volume of } CO_2 \text{ produced by user } (VCO_2) = (PaCO_2 * VA)/0.863$$

Here, a volume $VCO_2$ of carbon dioxide produced by the user may correspond to the amount of carbon dioxide produced by the user, $PaCO_2$ may denote a partial pressure of carbon dioxide in the user's arterial blood, VA may denote the amount of gas exchange in the user's alveoli and may be calculated based on ' $VA = VE-VD$ , VE may denote the amount of air inhaled into the user's lungs per minute (or the amount of air exhaled from the user's lungs per minute), and VD may denote a volume of air that does not take part in the gas exchange in the user's lungs.

According to an embodiment, the processor may measure a variation in the user's body fat by using at least one sensor (e.g., a bioelectrical impedance analysis (BIA) sensor).

According to an embodiment, when a variation in the user's body fat (or a variation in total calories according to a change in the user's body fat) is negative, the processor may determine that the user's body fat amount has increased.

According to an embodiment, when a variation in the user's body fat (or a variation in total calories according to a change in the user's body fat) is positive, the processor may determine that the user's body fat amount has decreased.

Here, the variation in the user's body fat may be calculated according to Equation 2 below, and the variation in calories according to the variation in the user's body fat may be calculated based on Equation 3 below (in this case, the user's body fat amount may be calculated based on ' and it is assumed that calories (user's body fat percentage) * (user's weight) alories (cal)).

variation in user's body fat = (user's body fat amount in previous state) - (user's body fat amount in current state)     [Equation 2]

variation in calories according to change in user's body fat = {(variation in user's body fat)*(calories corresponding to 1 g of body fat)}     [Equation 3]

For example, when the user's weight is 75 kilograms (kg), the user's body fat percentage in a previous state is 24.345 %, and the user's body fat percentage in a current state is 24.50 % (assuming that the calories corresponding to 1 gram of body fat amount are about 9 cal), a variation in calories according to a change in the user's body fat amount may be calculated as ' '. In other words, based on a $\{((24.345-24.50)/100)*75(kg)*9(cal/g)\}= -116.3(g)*9(cal/g) = -1046.7cal$ amount in the user's body has increased by an amount corresponding to '1046.7 cal'.

For example, when the user's weight is 75 kg, the user's body fat percentage in a previous state is 24.345 %, and the user's body fat percentage in a current state is 18.1875 % (assuming that the calories corresponding to 1 gram of body fat amount are about 9 cal), a variation in calories according to a change in the user's body fat amount may be calculated as '

$$\{((24.345-18.1875)/100)*75(kg)*9(cal/g)\}= 71.2(g)*9(cal/g)= 640.8cal$$

'. In other words, based on a result of the calculation, the processor may determine that body fat amount in the user's body has decreased by an amount corresponding to '640.8 cal'.

In operation 520, the processor may calculate the calorie burn amount of the user. In an embodiment, the processor may calculate the calorie burn amount of the user (kcal/minute) based on the amount of carbon dioxide produced by the user.

According to an embodiment, the processor may calculate the calorie burn amount of the user according to Equation 4 or Equation 5 based on the Weir formula (Equation 5 is an equation derived from Equation 4).

[Equation 4]

$$\text{calorie burn amount (kcal per minute)} = 3.94\ VO_2 + 1.11\ VCO_2$$

[Equation 5]

$$\text{calorie burn amount (kcal per minute)} = 3.94\ (VCO_2 / RQ) + 1.11\ VCO_2$$

Here, $VO_2$ represents a volume of oxygen consumed in liter per unit time (e.g., 1 minute), $VCO_2$ represents a volume of carbon dioxide produced in liter by the user per unit time (e.g., 1 minute), and RQ is a respiratory quotient, which may be a constant value of '0.86' or indicate a food quotient (the food quotient means ' total RQ/total calorie intake amount ').

In operation 530, the processor may calculate calorie intake amount of the user, based on the calorie burn amount of the user and the variation in the body fat. For example, the processor may autonomously calculate the user's calorie intake amount by using the calorie burn amount of the user and the variation in the body fat without a manual input from the user.

According to an embodiment, the processor may calculate the user's calorie intake amount according to Equation 6 below. In other words, the processor may autonomously calculate the user's calorie intake amount without the manual input from the user.

user's calorie intake amount (cal) = {(user's calorie burn amount) - (variation in calories according to change in user's body fat)}  [Equation 6]

For example, when the user's weight is 75 kg, the calorie burn amount of the user is '2000 cal', and the variation in calories according to the change in the user's body fat amount is '-1046.7 cal' (in this case, calories corresponding to 1 gram of body fat amount are assumed to be about 9 cal), the user's calorie intake amount may be '

$$\{2000-(-1046.7)\} = \{2000+1046.7\} = 3046.7cal$$

For example, when the user's weight is 75 kg, the calorie burn amount of the user is '2000 cal', and the variation in calories according to the change in the user's body fat amount is '+640.8 cal' (in this case, the calories corresponding to 1 gram of body fat amount are assumed to be about 9 cal), the user's calorie intake amount may be ' {2000-(640.8)} = 1359.2cal.

In operation 540, the processor may output user-customized healthcare information.

According to an embodiment, the processor may output user-customized healthcare information on a display of the electronic device based on the user's calorie intake amount calculated by the processor itself and user information (e.g., the user's diet and/or information about exercise preference, and information about the user's state or situation).

According to an embodiment, the user-customized healthcare information may include at least one of information about a diet recommended for the user and information about a type of exercise, an exercise time, or an exercise intensity recommended for the user.

In an embodiment, the processor may measure a metabolic rate of the user according to the user-customized healthcare information and store feedback information including the metabolic rate of the user. The processor may output user-customized healthcare information for a next cycle, based on the feedback information.

The electronic device according to the embodiment of the present disclosure may autonomously calculate the user's calorie intake amount and provide customized healthcare information, thereby improving a data error and user inconvenience caused by a manual input of biometric data by the user and providing an efficient user-customized healthcare system based on accurate biometric data.

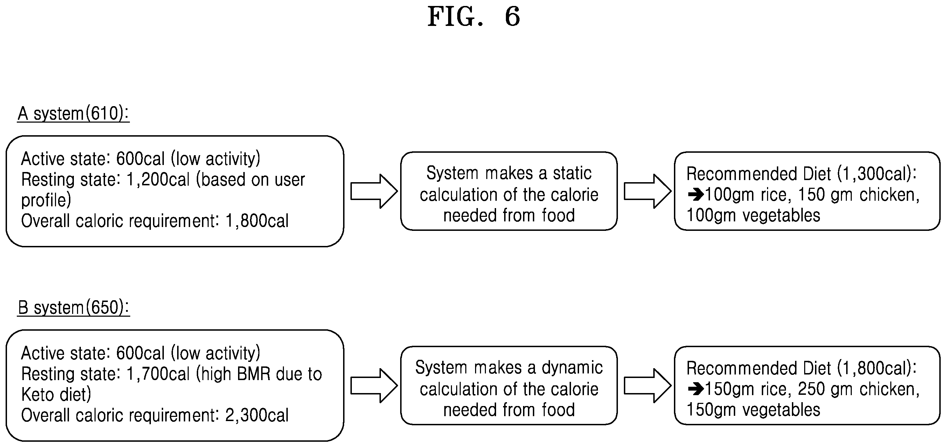

FIG. 6 illustrates an operation in which an electronic device outputs user-customized diet management information, according to an embodiment.

In detail, FIG. 6 illustrates an operation in which the electronic device 300 of FIG. 2 outputs user-customized diet management information to a user.

In FIG. 6, it is assumed that the user is a 24-year-old female, weighs 75 kg, is 5 feet (ft) 5 inches tall, does not prefer high-intensity activity, is currently in a resting state, has recently been on a ketogenic (keto) diet, and has set a goal of burning 500 cal for weight loss. It is also assumed that the user burns 600 cal in a low-intensity exercise state and 1200 cal in a resting state.

Referring to FIG. 6, a system A 610 may calculate an overall calorie burn amount of the user as 1800 cal, which is a sum of the calories (600 cal) burned in the low-intensity exercise state and the calories (1200 cal) burned in a resting state.

Therefore, the system A 610 may output (or recommend), to the user, information about a '1300-calorie' diet ( a diet of 100 grams of rice, 150 grams of chicken, and 100 grams of vegetables) to achieve a target calorie burn amount (500 cal) of the user for weight loss.

According to an embodiment of the present disclosure, a system B 650 may calculate the overall calorie burn amount of the user as '2300 cal', which is a sum of calories (600 cal) burned in a low-intensity exercise state and calories (1700 cal) burned in a resting state. In this case, the system B 650 may calculate the calorie burn amount of the user in a resting state as 1700 cal by taking into account a basal metabolic rate (BMR) increased due to the user's recent keto diet.

Therefore, the system B 610 may output (or recommend) to the user information about a '1800-calorie' diet ( a diet of 150 grams of rice, 250 grams of chicken, and 150 grams of vegetables) to achieve the target calorie burn amount (500 cal) of the user for weight loss.

According to the embodiment of the present disclosure, the electronic device (e.g., the system B 650) may recommend user-customized healthcare information to the user by taking into account not only basic information input by the user, but also a specific current situation or state of the user (e.g., the user being on a keto diet for weight loss).

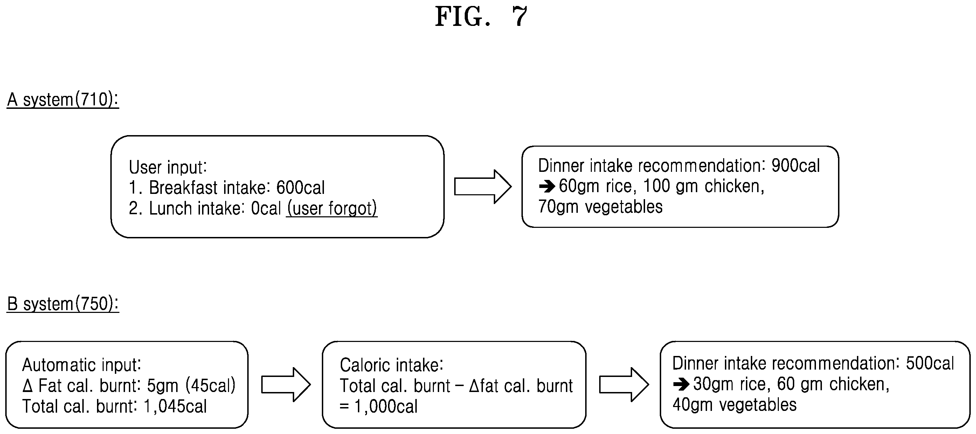

FIG. 7 illustrates an operation in which an electronic device calculates a user's calorie intake amount, according to an embodiment.

In detail, FIG. 7 illustrates an operation in which the electronic device 300 of FIG. 2 outputs user-customized diet management information to the user by autonomously calculating calorie intake amount of a user.

In FIG. 7, it is assumed that the user is a 24-year-old female, the user's weight is 75 kg, the user's height is 5 ft 5 inches, a target calorie burn amount of the user per day is 1500 cal, and the user's dinner menu is recommended based on the target calorie burn amount per day.

Referring to FIG. 7, a system A 710 is a system that manually receives the user's calorie intake amount for each meal from the user and outputs (or recommends) healthcare information to the user. The user may store calorie intake amount for breakfast (600 cal) in the system A 710, and forget to store calorie intake amount for lunch in the system A 710.

The system A 710 may determine calories to be consumed for dinner (900 cal) by subtracting only the user's breakfast calories (600 cal) from the target calorie burn amount per day (1500 cal). Accordingly, the system A 710 may output or recommend to the user information about a recommended dinner intake for consuming '900 cal' (e.g., a diet of 60 grams of rice, 100 grams of chicken, and 70 grams of vegetables).

A system B 750 is a system that autonomously calculates the user's calorie intake amount and outputs or recommends healthcare information to the user. The system B 750 may autonomously determine calories to be consumed for dinner (500 cal), based on a variation in calories (45 cal) due to burning of the user's body fat and the overall calorie burn amount of the user (1045 cal). Accordingly, the system B 750 may output or recommend to the user information about a recommended dinner intake for consuming '500 cal' (e.g., a diet of 30 grams of rice, 60 grams of chicken, and 40 grams of vegetables).

According to the embodiment of the present disclosure, the electronic device (e.g., the system B 750) may recommend user-customized healthcare information to the user by autonomously calculating a user's calorie intake amount based on the calorie burn amount of the user and a variation in the user's body fat (or a variation in calories according to a change in the user's body fat).

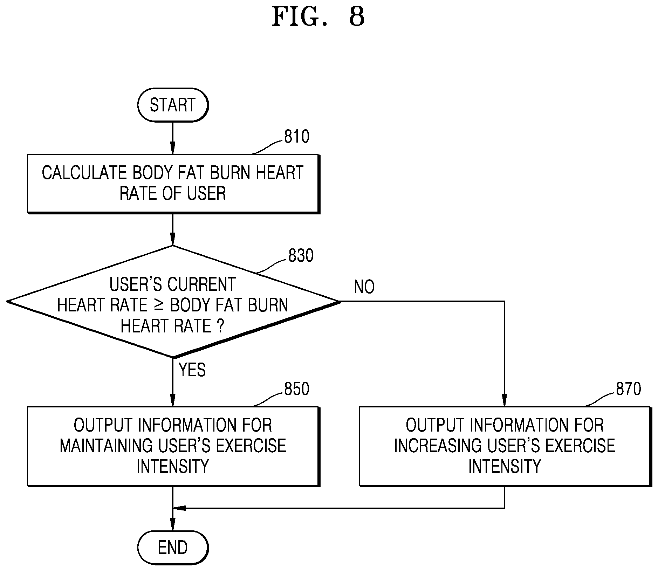

FIG. 8 illustrates an operation method of an electronic device, according to an embodiment.

In detail, FIG. 8 illustrates an operation method, performed by the processor of the electronic device 300 of FIG. 2, of outputting user-customized healthcare information based on information about a heart rate for burning the user's body fat.

Referring to FIG. 8, operations for outputting the user-customized healthcare information may include operations 810, 830, 850, and 870.

In operation 810, the processor may calculate a body fat burn heart rate of the user. In this specification, the body fat burn heart rate of the user may refer to the user's heart rate required to burn the user's body fat.

According to an embodiment, the processor may measure the calorie burn amount of the user, a maximum oxygen partial pressure within the user's body, and the user's current heart rate by using at least one sensor. In this case, the calorie burn amount of the user may be calculated based on the amount of carbon dioxide produced by the user, and the amount of carbon dioxide produced by the user may be measured based on Equation 1 in FIG. 5.

According to an embodiment, the processor may calculate the body fat burn heart rate of the user, based on the calorie burn amount of the user and the maximum oxygen partial pressure in the user's body.

In operation 830, the processor may identify whether the user's current heart rate is greater than or equal to the user's body fat burn heart rate.

According to an embodiment, when the user's current heart rate is greater than or equal to the heart rate for burning the user's body fat, the processor may perform operation 850.

According to an embodiment, when the user's current heart rate is less than the heart rate for burning the user's body fat, the processor may perform operation 870.

In operation 850, the processor may output information for maintaining the user's exercise intensity.

According to an embodiment, when the user's current heart rate is greater than or equal to the heart rate for burning the user's body fat, the processor may determine that a current exercise intensity is suitable for burning the user's body fat and output information for maintaining the user's current exercise intensity.

In operation 870, the processor may output information for increasing the user's exercise intensity.

According to an embodiment, when the user's current heart rate is less than the heart rate for burning the user's body fat, the processor may determine that the current exercise intensity is not suitable for burning the user's body fat, and output information for increasing the user's exercise intensity (e.g., information about the user's exercise speed, exercise duration, and the number of exercises.

For example, when the maximum oxygen partial pressure $VO_2MAX$ in the user's body is 185 beats per minute (bpm), and the user's body fat- burning heart rate calculated based on the user's current heart rate of 110 bpm is 130 bpm, the processor may determine that the user's current exercise intensity is insufficient to burn body fat, and output, onto the display of the electronic device, a message for recommending an increase in the exercise intensity to the user.

According to the embodiment of the present disclosure, the electronic device may recommend or output healthcare information to the user to maximize the burning of the user's body fat by using biometric information of the user(e.g., information about the calorie burn amount of the user and the maximum oxygen partial pressure in the user's body).

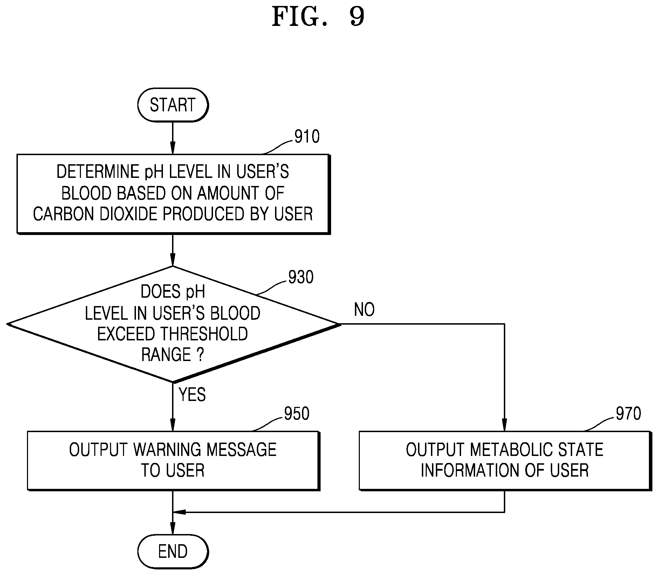

FIG. 9 illustrates an operation method of an electronic device, according to an embodiment.

In detail, FIG. 9 illustrates an operation method, performed by the processor of the electronic device 300 of FIG. 2, of outputting user-customized healthcare information by measuring a pH level in a user's blood based on the amount of carbon dioxide produced by the user. In an embodiment of the present disclosure, a 'pH level' may refer to the concentration of hydrogen ions in the user's blood.

Referring to FIG. 9, operations for outputting the user-customized healthcare information may include operations 910, 930, 950, and 970.

In operation 910, the processor may determine a pH level in the user's blood based on the amount of carbon dioxide produced by the user.

According to an embodiment, the processor may measure an amount of carbon dioxide generated by the user by using at least one sensor. For example, the processor may measure the amount of carbon dioxide generated by the user based on Equation 1 in FIG. 5 described above.

According to an embodiment, the processor may determine a pH level in the user's blood based on Equation 7 below. The following Equation 7 is based on the Henderson-Hasselbalch equation.

[Equation 7]

$$\text{pH level in user's blood} = 6.1 + \log_{10}(HCO_3/(0.0308 * PaCO_2))$$

Here, $HCO_3$[mEq/L] may denote the concentration of bicarbonate ions in the user's body, and $PaCO_2$[mmHg] may denote a partial pressure of carbon dioxide in the user's arterial blood (e.g., a carbon dioxide partial pressure in the arterial blood) (in this case, the partial pressure of carbon dioxide in the user's body may be proportional to the amount of carbon dioxide generated by the user).

According to an embodiment, the processor may calculate $HCO_3$[mEq/L] based on Equation 8 below. The following Equation 8 is based on the Winters equation.

[Equation 8]

$$HCO_3[mEq/L] = (PCO_2[mmHg] - 8 +/- 2)/ 1.5$$

Here, $HCO_3$[mEq/L] may denote the concentration of bicarbonate ions in the user's body, and $PCO_2$[mmHg] may denote a partial pressure of carbon dioxide in the user's body (in this case, the partial pressure of carbon dioxide in the user's body may be proportional to the amount of carbon dioxide generated by the user).

In operation 930, the processor may identify whether the pH level in the user's blood exceeds a threshold range.

According to an embodiment, when the pH level in the user's blood exceeds the threshold range, the processor may perform operation 950.

According to an embodiment, when the pH level in the user's blood does not exceed the threshold range, the processor may perform operation 970.

In operation 950, the processor may output a warning message to the user.

According to an embodiment, when the pH level in the user's blood exceeds the threshold range, the processor may output, to the user, a warning message recommending examination or treatment by determining that functions of the user's body (e.g., the lungs or kidneys) have been impaired.

According to an embodiment, the processor may calculate an average pH level of the user by repeatedly measuring a pH level in the user's blood over a specific period of time, and when there is a change in the average pH level in the user's blood, the processor may output a warning message to the user.

For example, when a pH level in the user's blood on day 1 is 7.38, a pH level in the user's blood on day 180 is 7.35, and a pH level in the user's blood on day 180 is 7.36, the processor may output a warning message to the user according to a decrease in an average pH level in the user's blood over a year.

In operation 970, the processor may output metabolic state information based on the pH level in the blood.

According to an embodiment, when the pH level in the user's blood does not exceed the threshold range, the processor may output, to the user, information about a metabolic state (e.g., an acidic state, an alkaline state, a normal state, etc.) according to the pH level in the user's blood.

According to an embodiment of the present disclosure, the electronic device may recommend or output healthcare information according to an internal state of the user's body based on the pH level in the user's blood measured noninvasively using the user's biometric information (e.g., the amount of carbon dioxide produced by the user).

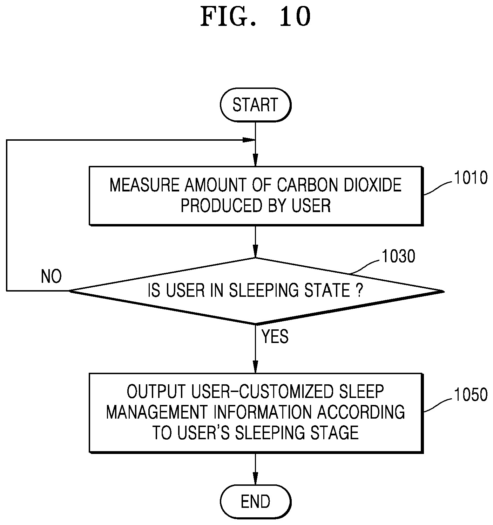

FIG. 10 illustrates an operation method of an electronic device, according to an embodiment.

In detail, FIG. 10 illustrates an operation method, performed by the processor of the electronic device 300 of FIG. 2, of outputting user-customized sleep management information by measuring the amount of carbon dioxide produced by a user.

According to an embodiment of the present disclosure, a sleep apnea state of the user may refer to a state in which a partial pressure of carbon dioxide in the user's blood increases due to the user stopping breathing or slowing down breathing for a certain period of time while sleeping.

Referring to FIG. 10, operations for outputting the user-customized healthcare information may include operations 1010, 1030, 1050, and 1070.

In operation 1010, the processor may measure the amount of carbon dioxide produced by the user. According to an embodiment, the processor may transcutaneously measure the amount of carbon dioxide produced by the user by using at least one sensor (e.g., an NDIR sensor).

In an embodiment, the processor may measure the amount of carbon dioxide produced by the user based on Equation 1 in FIG. 5 described above.

In operation 1030, the processor may identify whether the user is in a sleeping state. In an embodiment, because the amount of carbon dioxide generated by the user in a sleeping state tends to increase, the processor may identify whether the user is in a sleeping state based on the amount of carbon dioxide generated by the user.

According to an embodiment, when the amount of carbon dioxide generated by the user is below a target range, the processor may identify that the user is in a non-sleeping state (e.g., an active state).

According to an embodiment, when the amount of carbon dioxide generated by the user is above the target range, the processor may identify that the user is in a sleeping state and perform operation 1050.

In operation 1050, the processor may output user-customized sleep management information according to the user's sleeping stage. According to an embodiment of the present disclosure, the user-customized sleep management information may include at least one of sleep duration information, sleep period information, and sleeping posture information for the user to get better sleep.

For example, the processor may output sleeping posture information to the user to prevent the occurrence of sleep apnea in the user.

According to an embodiment, when the user is identified as being in a sleeping state, the processor may apply a weight to the amount of carbon dioxide generated by the user in the sleeping state (or the amount of carbon dioxide generated by the user in a non-sleeping state) in order to distinguish between the amounts of carbon dioxide respectively generated by the user while in the sleeping state and the non-sleeping state.

For example, there may be cases where the amount of carbon dioxide generated by the user increases not only when the user is in a sleeping state but also when the user is in a non-sleeping state (e.g., in an active state) due to an increase in the amount of metabolism in the body. Therefore, the processor may learn the user's biometric data (e.g., sleep cycle, the amount of carbon dioxide produced while the user is in each state, etc.), distinguish the user's sleeping state from the user's non-sleeping state based on the learned user's biometric data, and apply a weight to the amount of carbon dioxide produced according to each state.

According to an embodiment, the processor may output user-customized sleep management information for preventing sleep apnea based on a duration of the user's sleep apnea state measured while the user is in the sleeping state, the frequency of the user's sleep interruptions, and the user's current situation.

According to an embodiment, the user's sleeping stage may include a deep sleeping stage and a non-deep sleeping stage, and the deep sleeping stage and the non-deep sleeping stage may be distinguished based on the user's sleep cycle and the amount of carbon dioxide generated by the user in the sleeping state.

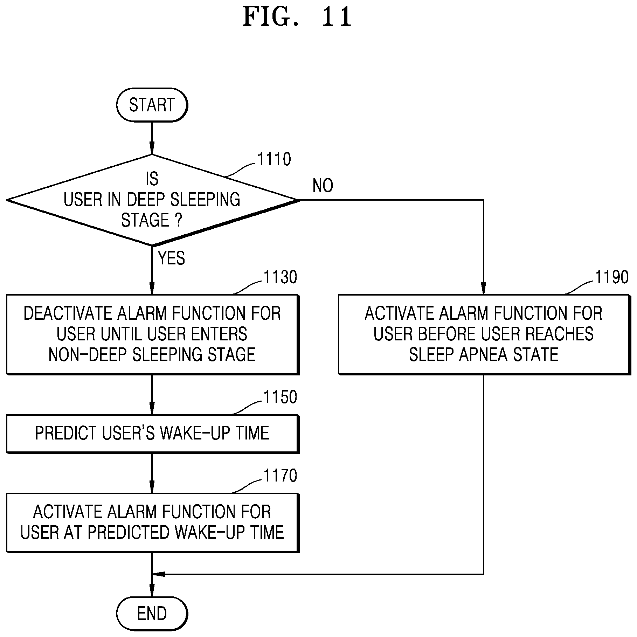

The operation in which the processor outputs user-customized sleep management information according to the user's sleeping stage is described in detail below with reference to FIG. 11.

Although not shown, in an embodiment, the processor may output (or recommend) user-customized sleep management information for a next cycle by taking into account user feedback information (e.g., the user's sleep state improvement, the user's preferences, or information about the user's context) on the output sleep management information.

FIG. 11 illustrates an operation method of an electronic device, according to an embodiment.

In detail, FIG. 11 illustrates an operation in which the processor of the electronic device 300 of FIG. 2 outputs user-customized sleep management information according to a user's sleeping stage.

Referring to FIG. 11, operations for outputting user-customized sleep management information for each sleeping stage may include operations 1110, 1130, 1150, 1170, and 1190.

In operation 1110, the processor may identify whether the user is in a deep sleeping stage.

According to an embodiment, the processor may measure, via at least one sensor, the user's sleep cycle, sleep period, or the amount of carbon dioxide generated by the user in a sleeping state.

According to an embodiment, the processor may determine that the user is in a sleep apnea state when the amount of carbon dioxide produced by the user in the sleeping state is greater than or equal to a threshold value. In this case, the sleep apnea state may refer to a state in which a partial pressure of carbon dioxide in the user's blood increases due to the user stopping breathing or slowing down breathing for a certain period of time while in the sleeping state.

According to an embodiment, the processor may identify whether the user is in a deep sleeping stage based on the user's sleep cycle and the amount of carbon dioxide generated by the user in the sleeping state.

According to an embodiment, if the user is identified as being in the deep sleeping stage, the processor may perform operation 1130.

According to an embodiment, when the user is in a non-deep sleeping stage, the processor may perform operation 1190.

In operation 1130, the processor may deactivate an alarm function for the user until the user enters the non-deep sleeping stage.

According to an embodiment, the processor may deactivate the alarm function for the user so as not to disturb the user's sleep until the user's sleeping stage changes to the non-deep sleeping stage.

In operation 1150, the processor may predict the user's wake-up time.

According to an embodiment, the processor may predict the user's wake-up time based on the user's sleep cycle, the user's sleep history information, and user profile information.

In operation 1170, the processor may activate a notification function or an alarm function for the user at the predicted user's wake-up time.

In operation 1190, the processor may activate an alarm function for the user before the user reaches a sleep apnea state.

According to an embodiment, the processor may store, for each sleep cycle, the amount of carbon dioxide produced by the user in a sleep apnea state.

In an embodiment, the processor may identify whether the user has reached a sleep apnea state, based on a result of comparison between the amount of carbon dioxide produced by the user in the sleeping state and the stored amount of carbon dioxide produced by the user in the sleep apnea state. Therefore, the processor may activate a notification function or an alarm function for the user before the user reaches a sleep apnea state according to the result of comparison.

According to an embodiment of the present disclosure, the electronic device may measure a sleeping stage based on the amount of carbon dioxide produced by the user in the sleeping state and recommend user-customized sleep management information to the user according to the user's sleeping stage, thereby improving the user's sleeping state and preventing risks (e.g., sleep apnea) that may occur while the user is in the sleeping state.

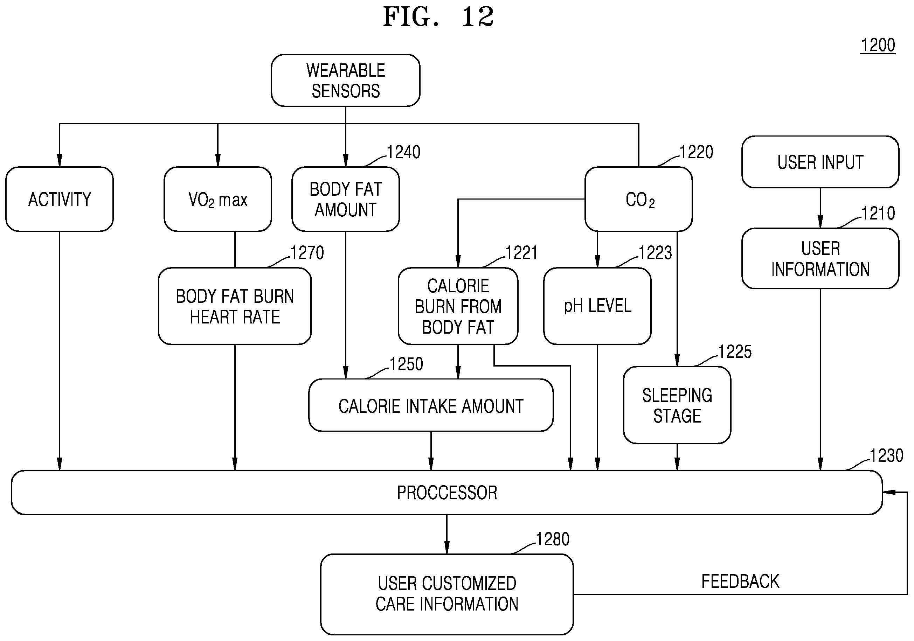

FIG. 12 is a block diagram of a system according to an embodiment.

In detail, FIG. 12 illustrates a system 1200 that outputs user-customized healthcare information or sleep management information by using biometric data of a user.

Referring to FIG. 12, according to an embodiment of the present disclosure, a processor 1230 of the system 1200 may output user-customized care information (e.g., user-customized healthcare information or user-customized sleep management information) 1280 by using various biometric data 1210, 1220, 1221, 1223, 1225, 1240, 1250, and 1270 of the user.

In an embodiment, the processor 1230 may receive user information (e.g., profile information, recent user context information, etc.) from the user and store the user information.

In an embodiment, the processor 1230 may measure the amount 1220 of carbon dioxide generated by the user by using at least one sensor (e.g., an NDIR sensor).

In an embodiment, based on the amount 1220 of carbon dioxide generated by the user, the processor 1230 may measure a calorie burn amount 1221 of the user and a pH level 1223 in the user's blood and determine a sleeping stage 1225 of the user.

In an embodiment, the processor 1230 may autonomously measure a calorie intake amount 1250 of the user without a user input, based on a body fat amount 1240 of the user (or a variation in the user's body fat) and the user's calorie burn amount 1221.

In an embodiment, the processor 1230 may measure a body fat burn heart rate 1270 of the user based on information about a maximum oxygen partial pressure $VO_2max$ in the user's body.

In an embodiment, the processor 1230 may recommend or output the user-customized care information 1280 (e.g., user-customized healthcare information or user-customized sleep management information) to the user by using the user information 1210 and information about the user's calorie burn amount 1221, the pH level 1223 in the user's blood, the user's sleeping stage 1225, the user's calorie intake amount 1250, the user's body fat burn heart rate 1250, and the user's activity.

In an embodiment, the processor 1230 may recommend or output user-customized care information for a next cycle by taking into account user feedback on the user-customized care information (e.g., user-customized healthcare information or user-customized sleep management information) 1280.

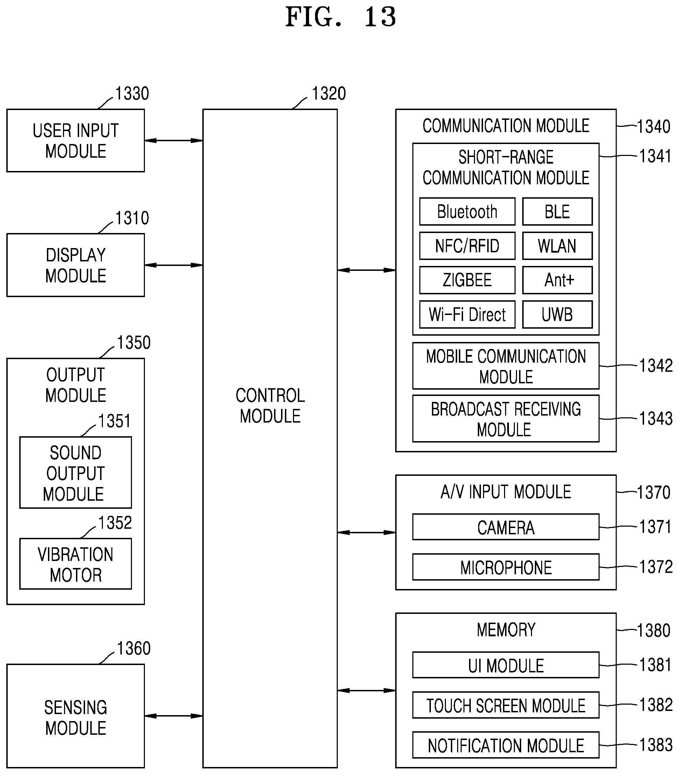

FIG. 13 illustrates a detailed configuration of an electronic device according to an embodiment.

In detail, FIG. 13 illustrates a detailed configuration of the electronic device 300 of FIG. 2 according to the embodiment. The sensing module 310, the touch input module 320, the display module 330, the communication module 340, and the control module 350 of FIG. 2 may respectively correspond to a sensing module 1360, a user input module 1330, a display module 1310, a communication module 1340, and a control module 1620 of FIG. 13. In addition, although not shown, the sensing module 1360 of FIG. 13 may be a module corresponding to the sensing module 310 and include at least one or a combination of the illuminance sensor 402, the biosensor 404, the tilt sensor 406, the position sensor 408, the proximity sensor 410, the geomagnetic sensor 412, the gyroscope sensor 414, the temperature/humidity sensor 416, the IR sensor 418, and the velocity/acceleration sensor 420 of FIG. 4.

Referring to FIG. 13, the electronic device according to an embodiment of the present disclosure may include the display module 1310, the control module 1320, the user input module 1330, the communication module 1340, an output module 1350, the sensing module 1360, an audio/video (A/V) input module 1370, and a memory 1380 of FIG. 13.

The display module 1310 may display and output information processed by the electronic device under the control of the control module 1320. In addition, the display module 1310 may display a graphical UI (GUI) on a screen.

According to an embodiment, the display module 1310 may output a notification signal related to control of the amount of a user's exercise.

According to an embodiment, according to control by the control module 1320, the display module 1310 may display a message related to whether a value contained in the past exercise record information has exceeded a value contained in current exercise record information.

Moreover, when the display 121 and a touch pad form a layered structure to construct a touch screen, the display 121 may be used as an input device as well as an output device. The display module 121 may include at least one of an LCD, a TFT-LCD, an OLED display, a flexible display, a 3D display, and an electrophoretic display. In addition, the electronic device may include two or more display modules 121 depending on a form of implementation of the electronic device. In this case, the two or more display modules 121 may be arranged to face each other by using a hinge.

The control module 1320 generally controls all operations of the electronic device. For example, the control module 1320 may control all operations of the display module 1310, the user input module 1330, the communication module 1340, the output module 1350, the A/V input module 1370, etc. by executing programs stored in the memory 1380.

In addition, the control module 1320 may execute an operating system (OS) and various applications stored in the memory 1380.

The control module 1320 may be implemented as a system on chip (SoC) that integrates a core (not shown) and a graphics processing unit (GPU) (not shown). In addition, the control module 1320 may include a single core, a dual core, a triple core, a quad core, and a number of cores equal to multiples thereof.

In an embodiment, the control module 1320 may calculate the calorie burn amount and the body fat amount of the user based on the measured amount of carbon dioxide generated by the user, and autonomously calculate the user's calorie intake amount by using the calorie burn amount and the body fat amount of the user.

In an embodiment, the control module 1320 may output user-customized healthcare information to the user based on the user's calorie burn amount, the user's body fat amount, and the user's calorie intake amount.

In an embodiment, the control module 1320 may output user-customized healthcare information according to a pH level in the user's blood.

In an embodiment, the control module 1320 may output user-customized healthcare information according to a body fat burn heart rate of the user.

In an embodiment, the control module 1320 may output user-customized sleep management information based on the user's sleeping stage.

The user input interface 1330 refers to a device via which the user inputs data for controlling the electronic device. For example, the user input interface 1330 may include, but is not limited to, a keypad, a dome switch, a touch pad (a capacitive overlay type, a resistive overlay type, an infrared beam type, a surface acoustic wave type, an integral strain gauge type, a piezoelectric type, etc.), a jog wheel, and a jog switch.

According to an embodiment, the user input module 1330 may receive, from the user, an input regarding user information (e.g., the user's profile, the user's recent context, the user's preferences, etc.).

According to an embodiment, the user input module 1330 may receive, from the user, an input for selecting one from a list including at least one piece of past exercise record information.

According to an embodiment, the user input module 1330 may receive, from the user, an input for starting, stopping, and ending exercise provision information.

The sound output interface 1351 may output audio data received from the communication interface 1340 or stored in the memory 1380. The sound output module 1351 also outputs sound signals associated with functions performed by the electronic device (e.g., a call signal reception sound, a message reception sound, and a notification sound). The sound output module 1351 may include a speaker, a buzzer, and the like.

A vibration motor 1352 may output a vibration signal. For example, the vibration motor 123 may output a vibration signal corresponding to an output of audio data or video data (e.g., a call signal reception sound, a message reception sound, etc.). In addition, the vibration motor 123 may also output a vibration signal when a touch is input to the touch screen.

The communication module 1340 may include one or more components that enable communication between the electronic device and an external server (e.g., an exercise record management server), between the electronic device and an external electronic device (e.g., a mobile phone, a smartphone, a PC, a laptop, etc.), or between external servers. For example, the communication module 1340 may include a short-range communication module 1341, a mobile communication module 1342, and a broadcast receiving module 1343.

The short-range communication module 1341 may include, but is not limited to, a Bluetooth communication unit, a BLE communication unit, an NFC module, a wireless local area network (WLAN) (or Wi-Fi) communication unit, a ZigBee communication unit, an Infrared Data Association (IrDA) communication unit, a Wi-Fi Direct (WFD) communication unit, an ultra-wideband (UWB) communication unit, an Ant+ communication unit, etc.

The mobile communication module 1342 transmits and receives a wireless signal to and from at least one of a base station, an external terminal, and a server on a mobile communication network. In this case, the wireless signal may be a voice call signal, a video call signal, or data in any one of various formats according to transmission and reception of a text/multimedia message.

The broadcast receiving module 1343 receives broadcast signals and/or broadcast-related information from the outside via a broadcast channel. The broadcasting channel may include a satellite channel and/or a terrestrial channel. Depending on an implementation example, the electronic device may not include the broadcast receiving module 1343.

The sensing module 1360 may include at least one or a combination of an illuminance sensor, a biosensor, a tilt sensor, a position sensor, a proximity sensor, a geomagnetic sensor, a gyroscope sensor, a temperature/humidity sensor, an IR sensor, and a velocity/acceleration sensor. In addition to the stated types of sensors, various sensors may be included in the sensing module 1360.

In an embodiment, the biosensor may sense information related to the user's body composition and biosignals. The biosensor may include, for example, at least one of a heart rate sensor, a blood glucose sensor, a blood pressure sensor, a sweat sensor, a body temperature sensor, and an iris sensor.

For example, the biosensor may measure the calorie burn amount of the user, the user's body fat percentage, the user's body fat amount, the user's metabolic rate, the user's sleep cycle or sleep period.

For example, the biosensor may measure a partial pressure of oxygen in the user's body (e.g., a maximum oxygen partial pressure $VO_2MAX$ )or a partial pressure of carbon dioxide in the user's body.

For example, the biosensor may measure a pH level in the user's blood by using the amount of carbon dioxide produced by the user.

For example, the position sensor may sense contextual information of the user based on the user's location.

For example, the IR sensor includes an NDIR sensor and may perform real-time transcutaneous measurement of the amount of carbon dioxide produced by the user.

The A/V input module 1370 is for inputting audio signals or video signals, and may include a camera 1371 and a microphone 1372. The camera 1371 may obtain image frames such as still images or moving images via an image sensor in a video call mode or shooting mode. An image captured via the image sensor may be processed by the control module 1320 or a separate image processing module (not shown).

The image frames processed by the camera 1371 may be stored in the memory 1380 or transmitted to the outside via the communication module 1340. The camera 1371 may be two or more cameras depending on a configuration of the terminal.

The microphone 1372 receives an external sound signal and process the external sound signal into electrical audio data. For example, the microphone 1372 may receive a sound signal from an external device or a speaker. The microphone 1372 may use various noise removal algorithms to remove noise that occurs in the process of receiving an external sound signal.

The memory 1380 may store a program for processing and controlling the control module 1320, and store input/output data (e.g., a plurality of menus, a plurality of first-layer sub-menus corresponding to each of the plurality of menus, a plurality of second-layer sub-menus corresponding to each of the plurality of first-layer sub-menus, etc.).

The memory 1380 may include at least one type of storage medium among a hard disk-type memory, a multimedia card micro-type memory, a card-type memory (e.g., a Secure Digital (SD) card or an eXtreme Digital (XD) memory), random access memory (RAM), static RAM (SRAM), read-only memory (ROM), electrically erasable programmable ROM (EEPROM), PROM, a magnetic memory, a magnetic disc, and an optical disc. In addition, the electronic device may operate a web storage or cloud server that performs a storage function of the memory 1380 on the Internet.

Programs stored in the memory 1380 may be categorized into a plurality of modules according to their functions, for example, a UI module 1381, a touch screen module 1382, a notification module 1383, etc.

The UI module 1381 may provide specialized UI, GUI, etc. that are linked to the electronic device for each application. The touch screen module 1382 may detect a touch gesture on the user's touch screen and transmit information about the touch gesture to the control module 1320. According to an embodiment, the touch screen module 1382 may recognize and analyze a touch code. The touch screen module 1382 may also be configured as separate hardware including a controller.

Various sensors may be provided inside or near the touch screen to detect a touch or proximity touch on the touch screen. A tactile sensor is an example of a sensor for detecting a touch on the touch screen. The tactile sensor is a sensor that detects a contact with a specific object to a degree or greater than that felt by a human. The tactile sensor may detect various types of information such as the roughness of a contact surface, the hardness of an object in contact, and the temperature of a contact point.

In addition, a proximity sensor is an example of a sensor for detecting a touch on a touch screen.

The proximity sensor is a sensor that detects the presence or absence of an object approaching a predetermined detection surface or nearby objects without mechanical contact by using a force from an electromagnetic field or IR light. Examples of proximity sensors include a transmission-type photoelectric sensor, a direct reflection-type photoelectric sensor, a mirror reflection-type photoelectric sensor, a high-frequency oscillation-type proximity sensor, a capacitive proximity sensor, a magnetic proximity sensor, and an IR proximity sensor. User touch gestures may include tap, touch & hold, double tap, drag, flick, swipe, etc.

The notification module 1383 may generate a signal for notifying the occurrence of an event in the electronic device. Examples of events occurring in the electronic device include reception of a call signal, reception of a message, input of a key signal, and notification of a schedule. The notification module 1383 may output a notification signal in the form of a video signal via the display module 1310, output a notification signal in the form of an audio signal via the sound output module 1351, and output a notification signal in the form of a vibration signal via the vibration motor 1352.

In an embodiment, the notification module 1383 may output a notification, warning, or alarm signal to the user according to user-customized care information. For example, when a pH level in the user's blood exceeds a threshold range, the notification module 1383 may output a warning signal to the user. For example, when a sleeping user is identified as being in a sleep apnea state (e.g., when the amount of carbon dioxide generated by the user is greater than or equal to a threshold value), the notification module 1383 may output an alarm signal to the user.

The names of the components of the electronic device may vary. In addition, the electronic device according to the present disclosure may be configured with at least one of the components, and may not include some of the components but further include other components.

A method according to an embodiment of the present disclosure may be implemented in the form of program commands that may be performed by various types of computers, and may be recorded on computer-readable recording media. The computer-readable recording media may include program commands, data files, data structures, etc. either alone or in combination. The program commands recorded on the media may be designed and configured specially for the present disclosure or may be known to and be usable by those of ordinary skill in the art of computer software. Examples of the computer-readable recording media include magnetic media such as hard disks, floppy disks, and magnetic tapes, optical media such as compact disk ROM (CD-ROM) and digital versatile disks (DVDs), magneto-optical media such as floptical disks, and hardware devices that are specially configured to store and perform program commands, such as ROM, RAM, flash memory, etc. Examples of program commands include not only machine code such as that created by a compiler but also high-level language code that may be executed by a computer using an interpreter or the like.

An embodiment of the disclosure and all functional operations described herein may be implemented within a digital electronic circuit, or in computer software, firmware, or hardware, including the structures disclosed herein and their equivalent structures, or in any combination of one or more of these.

An electronic device according to an embodiment includes at least one sensor, a memory storing one or more instructions, and one or more processors, and the one or more processors may be configured to execute the one or more instructions to measure, by using the at least one sensor, an amount of carbon dioxide generated by a user and a variation in body fat of the user, calculate a calorie burn amount of the user, based on the amount of carbon dioxide generated by the user, calculate a calorie intake amount of the user based on the variation in the body fat of the user and the calorie burn amount of the user, and output user-customized healthcare information, based on the variation in the body fat of the user, the calorie burn amount of the user, and the calorie intake amount of the user.

According to an embodiment, the one or more processors may be configured to measure a metabolic rate of the user according to the user-customized healthcare information, store feedback information including the metabolic rate of the user, and output user-customized healthcare information, based on the feedback information.

According to an embodiment, the user-customized healthcare information may be configured to be output to the user in real time as information including at least one of information about a diet recommended for the user and information about a type of exercise, an exercise time, or an exercise intensity recommended for the user.

According to an embodiment, the one or more processors may be further configured to identify, by using the at least one sensor, whether a state of the user is a sleeping state, and apply a weight to the measured amount of carbon dioxide when the state of the user is a sleeping state.

According to an embodiment, the one or more processors may be further configured to identify whether the amount of carbon dioxide is above a target range, determine the state of the user as being a sleeping state when the amount of carbon dioxide is above the target range, and provide an alarm to the user according to a sleeping stage of the user.

According to an embodiment, the one or more processors may be further configured to, when the sleeping stage is a non-deep sleeping stage, activate an alarm function for the user before the user reaches a sleep apnea state, and when the sleeping stage is a deep sleeping stage, deactivate the alarm function for the user before the user enters the non-deep sleeping stage.

In an embodiment, the one or more processors may be further configured to identify a pH level in the user's blood based on the measured amount of carbon dioxide.

According to an embodiment, the one or more processors may be further configured to provide a warning to the user when the pH level in the user's blood exceeds a threshold range, and output to the user a metabolic state of the user according to the pH level in the user's blood when the pH level in the user's blood does not exceed the threshold range.

According to an embodiment, the at least one sensor further comprises an NDIR sensor, and the one or more processors may be configured to transcutaneously measure an amount of carbon dioxide generated by the user by using the NDIR sensor.

In an embodiment, the one or more processors may be further configured to calculate a heart rate of the user required to reach a maximum amount of oxygen consumed by the user per unit time, and output the user-customized healthcare information based on the heart rate of the user.

An operation method of an electronic device, according to an embodiment, may include measuring an amount of carbon dioxide generated by a user and a variation in body fat of the user, calculating a calorie burn amount of the user, based on the amount of carbon dioxide, calculating a calorie intake amount of the user based on the variation in the body fat of the user and the calorie burn amount of the user, and outputting user-customized healthcare information, based on the calorie burn amount of the user, the variation in the body fat of the user, and the calorie intake amount of the user.

According to an embodiment, the operation method of the electronic device may include measuring a metabolic rate of the user according to the user-customized healthcare information, storing feedback information including the metabolic rate of the user, and outputting subsequent user-customized healthcare information, based on the feedback information.

According to an embodiment, in the operation method of the electronic device, the user-customized healthcare information may be configured to be output to the user in real time as information including at least one of information about a diet recommended for the user and information about a type of exercise, an exercise time, or an exercise intensity recommended for the user.

According to an embodiment, the operation method of the electronic device may further include identifying whether a state of the user is a sleeping state, and when the state of the user is a sleeping state, applying a weight to the measured amount of carbon dioxide.

According to an embodiment, the operation method of the electronic device may further include identifying whether the amount of carbon dioxide is above a target range, determining the state of the user as being a sleeping state when the amount of carbon dioxide is above the target range, and providing an alarm to the user according to a sleeping stage of the user.

According to an embodiment, the operation method of the electronic device may further include, when the sleeping stage of the user is a non-deep sleeping stage, activating an alarm function for the user before the user reaches a sleep apnea state, and when the sleeping stage of the user is a deep sleeping stage, deactivating the alarm function for the user before the user enters the non-deep sleeping stage.

According to an embodiment, the operation method of the electronic device may further include identifying a pH level in the user's blood based on the measured amount of carbon dioxide.

According to an embodiment, the operation method of the electronic device may further include providing a warning to the user when the pH level in the user's blood exceeds a threshold range, and outputting a metabolic state of the user according to the pH level in the user's blood to the user when the pH level in the user's blood does not exceed the threshold range.

According to an embodiment, the operation method of the electronic device may further include calculating a heart rate of the user required to reach a maximum amount of oxygen consumed by the user per unit time, and outputting the user-customized healthcare information based on the heart rate of the user.

The operation method of the electronic device, according to an embodiment, may be implemented in the form of program commands that may be performed by various types of computers, and may be recorded on computer-readable recording media. Furthermore, the embodiments of the present disclosure may be implemented in the form of recording media including instructions executable by a computer, such as program modules executed by the computer. The computer-readable recording media may be any available media that are accessible by the computer, and examples thereof may include both volatile and non-volatile media and both detachable and non-detachable media. Furthermore, the computer-readable recording media may include computer storage media and communication media. The computer storage media include both volatile and nonvolatile, removable and non-removable media implemented using any method or technology for storage of information such as computer-readable instructions, data structures, program modules, or other data. The communication media may typically include computer-readable instructions, data structures, program modules, or other data in a modulated data signal, such as program modules.

The computer-readable recording media may include program commands, data files, data structures, etc. either alone or in combination. The program commands recorded on the computer-readable recording media may be designed and configured specially for the present disclosure or may be known to and be usable by those of ordinary skill in the art of computer software. Examples of the computer-readable recording media include magnetic media such as hard disks, floppy disks, and magnetic tapes, optical media such as compact disk ROM (CD-ROM) and digital versatile disks (DVDs), magneto-optical media such as floptical disks, and hardware devices that are specially configured to store and perform program commands, such as ROM, RAM, flash memory, etc. Examples of program commands include not only machine code such as that created by a compiler but also high-level language code that may be executed by a computer using an interpreter or the like.

In addition, at least one of an operation method of a display device and an operation method of an electronic device according to disclosed embodiments may be included in a computer program product when provided. The computer program product may be traded, as a product, between a seller and a buyer.

The computer program product may include a software program and a computer-readable storage medium having the software program stored thereon. For example, the computer program product may include a product (e.g., a downloadable application) in the form of a software program electronically distributed by a manufacturer of an electronic device or through an electronic market (e.g., Google Play Store ™, and App Store ™). For such electronic distribution, at least a part of the software program may be stored on the storage medium or may be temporarily generated. In this case, the storage medium may be a storage medium of a server of the manufacturer, a server of the electronic market, or a relay server for temporarily storing the software program.

The above description of the present disclosure is provided for illustration, and it will be understood by one of ordinary skill in the art that changes in form and details may be readily made therein without departing from technical idea or essential features of the present disclosure. Therefore, the above-described embodiments and all aspects thereof are merely examples and are not limiting.

The scope of the present disclosure is defined not by the detailed description thereof but by the following claims, and all the changes or modifications within the meaning and scope of the appended claims and their equivalents should be construed as being included in the scope of the present disclosure.

**Claims**

1. An electronic device comprising:

   at least one sensor;
   a memory storing one or more instructions; and
   one or more processors, wherein the one or more processors are configured to execute the one or more instructions to
   measure, by using the at least one sensor, an amount of carbon dioxide generated by a user and a variation in body fat of the user,
   calculate a calorie burn amount of the user, based on the amount of carbon dioxide generated by the user,
   calculate a calorie intake amount of the user, based on the variation in the body fat of the user and the calorie burn amount of the user, and
   output user-customized healthcare information, based on the variation in the body fat of the user, the calorie burn amount of the user, and the calorie intake amount of the user.

2. The electronic device of claim 1, wherein

   the one or more processors are further configured to
   measure a metabolic rate of the user according to the user-customized healthcare information,
   store feedback information including the metabolic rate of the user, and
   output user-customized healthcare information, based on the feedback information.

3. The electronic device of claim 1, wherein

   the one or more processors are further configured to
   identify, by using the at least one sensor, whether a state of the user is a sleeping state, and
   apply a weight to the measured amount of carbon dioxide when the state of the user is a sleeping state.

4. The electronic device of claim 3, wherein

   the one or more processors are further configured to
   identify whether the amount of carbon dioxide is above a target range,
   determine the state of the user as being a sleeping state when the amount of carbon dioxide is above the target range, and
   provide an alarm to the user according to a sleeping stage of the user.

5. The electronic device of claim 4, wherein

   the one or more processors are further configured to,
   when the sleeping stage is a non-deep sleeping stage, activate an alarm function for the user before the user reaches a sleep apnea state, and
   when the sleeping stage is a deep sleeping stage, deactivate the alarm function for the user before the user enters the non-deep sleeping stage.

6. The electronic device of claim 1, wherein

   the one or more processors are further configured to

identify a pH level in the user's blood, based on the measured amount of carbon dioxide,

provide a warning to the user when the pH level in the user's blood exceeds a threshold range, and

output, to the user, a metabolic state of the user according to the pH level in the user's blood when the pH level in the user's blood does not exceed the threshold range.

7. The electronic device of claim 1, wherein

the one or more processors are further configured to

calculate a heart rate of the user required to reach a maximum amount of oxygen consumed by the user per unit time, and

output the user-customized healthcare information, based on the heart rate of the user.

8. An operation method of an electronic device, the operation method comprising:

measuring an amount of carbon dioxide generated by a user and a variation in body fat of the user;

calculating a calorie burn amount of the user, based on the amount of carbon dioxide;

calculating a calorie intake amount of the user, based on the variation in the body fat of the user and the calorie burn amount of the user; and

outputting user-customized healthcare information, based on the calorie burn amount of the user, the variation in the body fat of the user, and the calorie intake amount of the user.

9. The operation method of claim 8, comprising:

measuring a metabolic rate of the user according to the user-customized healthcare information;

storing feedback information including the metabolic rate of the user; and

outputting subsequent user-customized healthcare information, based on the feedback information.

10. The operation method of claim 8, further comprising:

identifying whether a state of the user is a sleeping state; and

when the state of the user is a sleeping state, applying a weight to the measured amount of carbon dioxide.

11. The operation method of claim 10, further comprising:

identifying whether the amount of carbon dioxide is above a target range;

determining the state of the user as being a sleeping state when the amount of carbon dioxide is above the target range; and

providing an alarm to the user according to a sleeping stage of the user.

12. The operation method of claim 11, further comprising:

when the sleeping stage of the user is a non-deep sleeping stage, activating an alarm function for the user before the user reaches a sleep apnea state; and

when the sleeping stage of the user is a deep sleeping stage, deactivating the alarm function for the user before the user enters the non-deep sleeping stage.

13. The operation method of claim 8, further comprising:

identifying a pH level in the user's blood based on the measured amount of carbon dioxide;

providing a warning to the user when the pH level in the user's blood exceeds a threshold range; and

outputting, to the user, a metabolic state of the user according to the pH level in the user's blood when the pH level in the user's blood does not exceed the threshold range.

14. The operation method of claim 8, further comprising:

calculating a heart rate of the user required to reach a maximum amount of oxygen consumed by the user per unit time; and

outputting the user-customized healthcare information, based on the heart rate of the user.

15. A computer-readable storage medium having recorded thereon a program for performing the operation method of any one of claims 8 to 14 on a computer.

# FIG. 1

100

10

# FIG. 2

300

310 SENSING MODULE

350 CONTROL MODULE

330 DISPLAY MODULE

320 TOUCH INPUT MODULE

340 COMMUNICATION MODULE

# FIG. 3A

# FIG. 3B

# FIG. 4

| | 310 |
|---|---|
| 402 — ILLUMINANCE SENSOR | GEOMAGNETIC SENSOR — 412 |
| 404 — BIO SENSOR | GYROSCOPE SENSOR — 414 |
| 406 — TILT SENSOR | TEMPERATURE /HUMIDITY SENSOR — 416 |
| 408 — POSITION SENSOR | INFRARED SENSOR — 418 |
| 410 — PROXIMITY SENSOR | VELOCITY /ACCELERATION SENSOR — 420 |

# FIG. 5

START

MEASURE AMOUNT OF CARBON DIOXIDE PRODUCED BY USER AND VARIATION IN BODY FAT —510

CALCULATE CALORIE BURN AMOUNT OF USER —520

CALCULATE CALORIE INTAKE AMOUNT OF USER, BASED ON CALORIE BURN AMOUNT OF USER AND VARIATION IN BODY FAT —530

OUTPUT USER-CUSTOMIZED HEALTHCARE INFORMATION —540

END

# FIG. 6

EP 4 586 262 A1

A system(610):

| Active state: 600cal (low activity)<br>Resting state: 1,200cal (based on user profile)<br>Overall caloric requirement: 1,800cal | ⇒ | System makes a static calculation of the calorie needed from food | ⇒ | Recommended Diet (1,300cal):<br>➔100gm rice, 150 gm chicken, 100gm vegetables |

B system(650):

| Active state: 600cal (low activity)<br>Resting state: 1,700cal (high BMR due to Keto diet)<br>Overall caloric requirement: 2,300cal | ⇒ | System makes a dynamic calculation of the calorie needed from food | ⇒ | Recommended Diet (1,800cal):<br>➔150gm rice, 250 gm chicken, 150gm vegetables |

# FIG. 7

A system(710):

User input:
1. Breakfast intake: 600cal
2. Lunch intake: 0cal (user forgot)

⇨

Dinner intake recommendation: 900cal
➔ 60gm rice, 100 gm chicken,
70gm vegetables

B system(750):

Automatic input:
Δ Fat cal. burnt: 5gm (45cal)
Total cal. burnt: 1,045cal

⇨

Caloric intake:
Total cal. burnt − Δfat cal. burnt
= 1,000cal

⇨

Dinner intake recommendation: 500cal
➔ 30gm rice, 60 gm chicken,
40gm vegetables

# FIG. 8

```
            ┌─────────┐
            │  START  │
            └─────────┘
                 │
                 ▼                        810
   ┌──────────────────────────────┐
   │  CALCULATE BODY FAT BURN HEART │
   │        RATE OF USER            │
   └──────────────────────────────┘
                 │
                 ▼                        830
          ╱────────────────╲                    NO
         ╱  USER'S CURRENT   ╲──────────────────────┐
         ╲ HEART RATE ≥ BODY  ╱                      │
          ╲ FAT BURN          ╱                      │
           ╲ HEART RATE ?    ╱                       │
            ╲───────────────╱                        │
                 │ YES                               │
                 ▼              850                   ▼              870
   ┌──────────────────────────────┐    ┌──────────────────────────────┐
   │   OUTPUT INFORMATION FOR      │    │   OUTPUT INFORMATION FOR       │
   │  MAINTAINING USER'S EXERCISE  │    │  INCREASING USER'S EXERCISE    │
   │         INTENSITY             │    │         INTENSITY              │
   └──────────────────────────────┘    └──────────────────────────────┘
                 │                                    │
                 ▼◄───────────────────────────────────┘
            ┌─────────┐
            │   END   │
            └─────────┘
```

# FIG. 9

START

910
DETERMINE pH LEVEL IN USER'S BLOOD BASED ON AMOUNT OF CARBON DIOXIDE PRODUCED BY USER

930
DOES pH LEVEL IN USER'S BLOOD EXCEED THRESHOLD RANGE ?

NO

YES

950
OUTPUT WARNING MESSAGE TO USER

970
OUTPUT METABOLIC STATE INFORMATION OF USER

END

# FIG. 10

START

MEASURE AMOUNT OF CARBON DIOXIDE
PRODUCED BY USER — 1010

IS USER IN SLEEPING STATE ? — 1030

NO

YES

OUTPUT USER-CUSTOMIZED SLEEP
MANAGEMENT INFORMATION ACCORDING
TO USER'S SLEEPING STAGE — 1050

END

# FIG. 11

```
        ( START )
            │
            ▼
         ╱─────────╲          1110
        ╱    IS     ╲         NO
  ◄────╱ USER IN DEEP ╲──────────────────┐
        ╲  SLEEPING   ╱                   │
         ╲  STAGE ?  ╱                    │
          ╲─────────╱                     │
            │ YES                         │
            ▼                             ▼
┌──────────────────────────┐ 1130  ┌──────────────────────────┐ 1190
│ DEACTIVATE ALARM FUNCTION│       │  ACTIVATE ALARM FUNCTION │
│  FOR USER UNTIL USER     │       │  FOR USER BEFORE USER    │
│  ENTERS NON-DEEP         │       │  REACHES SLEEP APNEA     │
│  SLEEPING STAGE          │       │  STATE                   │
└──────────────────────────┘       └──────────────────────────┘
            │                             │
            ▼                             │
┌──────────────────────────┐ 1150        │
│ PREDICT USER'S WAKE-UP   │             │
│ TIME                     │             │
└──────────────────────────┘             │
            │                             │
            ▼                             │
┌──────────────────────────┐ 1170        │
│  ACTIVATE ALARM FUNCTION │             │
│  FOR USER AT PREDICTED   │             │
│  WAKE-UP TIME            │             │
└──────────────────────────┘             │
            │◄────────────────────────────┘
            ▼
        ( END )
```

# FIG. 12

EP 4 586 262 A1

# FIG. 13

USER INPUT MODULE — 1330

DISPLAY MODULE — 1310

OUTPUT MODULE — 1350

SOUND OUTPUT MODULE — 1351

VIBRATION MOTOR — 1352

SENSING MODULE — 1360

CONTROL MODULE — 1320

COMMUNICATION MODULE — 1340

SHORT-RANGE COMMUNICATION MODULE — 1341

| Bluetooth | BLE |
|-----------|-----|
| NFC/RFID | WLAN |
| ZIGBEE | Ant+ |
| Wi-Fi Direct | UWB |

MOBILE COMMUNICATION MODULE — 1342

BROADCAST RECEIVING MODULE — 1343

A/V INPUT MODULE — 1370

CAMERA — 1371

MICROPHONE — 1372

MEMORY — 1380

UI MODULE — 1381

TOUCH SCREEN MODULE — 1382

NOTIFICATION MODULE — 1383

# INTERNATIONAL SEARCH REPORT

| International application No. |
| --- |
| **PCT/KR2023/013452** |

## A. CLASSIFICATION OF SUBJECT MATTER

**G16H 20/00**(2018.01)i; **G16H 50/20**(2018.01)i; **G16H 20/60**(2018.01)i; **G16H 20/30**(2018.01)i; **A63B 24/00**(2006.01)i; **A61B 5/00**(2006.01)i; **A61B 5/083**(2006.01)i; **A61B 5/145**(2006.01)i; **A61B 5/021**(2006.01)i

According to International Patent Classification (IPC) or to both national classification and IPC

## B. FIELDS SEARCHED

Minimum documentation searched (classification system followed by classification symbols)

G16H 20/00(2018.01); A61B 5/00(2006.01); A61J 15/00(2006.01); G06Q 50/10(2012.01); G06Q 50/22(2012.01); G16H 20/30(2018.01); G16H 20/70(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 센서(sensor), 이산화탄소(carbon dioxide), 칼로리(calorie), 소모량(consumption), 변화량(variance), 맞춤형(customized), 건강(health)

## C. DOCUMENTS CONSIDERED TO BE RELEVANT

| Category* | Citation of document, with indication, where appropriate, of the relevant passages | Relevant to claim No. |
| --- | --- | --- |
| Y | KR 10-2197102 B1 (JUDORI) 30 December 2020 (2020-12-30)<br>See paragraphs [0011], [0028]-[0048], [0075] and [0086], claims 1-2 and figures 1-3. | 1-15 |
| Y | JP 2020-512919 A (ART MEDICAL LTD.) 30 April 2020 (2020-04-30)<br>See paragraphs [0119]-[0122] and claims 1-2, 7 and 22. | 1-15 |
| Y | KR 10-2020-0132198 A (ENPLUG CO., LTD.) 25 November 2020 (2020-11-25)<br>See paragraph [0064] and claims 3-4. | 3-5,7,10-12,14 |
| A | WO 2013-038959 A1 (NTT DOCOMO, INC. et al.) 21 March 2013 (2013-03-21)<br>See entire document. | 1-15 |
| A | KR 10-2021-0144967 A (YOUN, Joung Hwan) 01 December 2021 (2021-12-01)<br>See entire document. | 1-15 |

☐ Further documents are listed in the continuation of Box C.  ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **11 December 2023** | **11 December 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/013452**

| Patent document cited in search report | | | Publication date (day/month/year) | Patent family member(s) | | | Publication date (day/month/year) |
|---|---|---|---|---|---|---|---|
| KR | 10-2197102 | B1 | 30 December 2020 | None | | | |
| JP | 2020-512919 | A | 30 April 2020 | AU | 2017-408431 | A1 | 14 November 2019 |
| | | | | AU | 2017-408431 | B2 | 31 August 2023 |
| | | | | BR | 112019020663 | A2 | 14 July 2020 |
| | | | | CA | 3057964 | A1 | 11 October 2018 |
| | | | | CN | 110692108 | A | 14 January 2020 |
| | | | | EP | 3607556 | A1 | 12 February 2020 |
| | | | | EP | 3607556 | B1 | 01 June 2022 |
| | | | | JP | 2022-177187 | A | 30 November 2022 |
| | | | | US | 10898413 | B2 | 26 January 2021 |
| | | | | US | 11185474 | B2 | 30 November 2021 |
| | | | | US | 11364180 | B2 | 21 June 2022 |
| | | | | US | 2020-0222286 | A1 | 16 July 2020 |
| | | | | US | 2021-0169744 | A1 | 10 June 2021 |
| | | | | US | 2022-0079845 | A1 | 17 March 2022 |
| | | | | WO | 2018-185738 | A1 | 11 October 2018 |
| KR | 10-2020-0132198 | A | 25 November 2020 | KR | 10-2212200 | B1 | 04 February 2021 |
| WO | 2013-038959 | A1 | 21 March 2013 | CN | 103503016 | A | 08 January 2014 |
| | | | | CN | 103503016 | B | 15 February 2017 |
| | | | | EP | 2657899 | A1 | 30 October 2013 |
| | | | | EP | 2657899 | B1 | 04 March 2020 |
| | | | | JP | 5696222 | B2 | 08 April 2015 |
| | | | | US | 2013-0288208 | A1 | 31 October 2013 |
| | | | | US | 9183757 | B2 | 10 November 2015 |
| | | | | WO | 2013-038959 | A1 | 26 March 2015 |
| KR | 10-2021-0144967 | A | 01 December 2021 | KR | 10-2359416 | B1 | 07 February 2022 |

Form PCT/ISA/210 (patent family annex) (July 2022)